(19) Europäisches Patentamt
European Patent Office
Office européen des brevets

(11) EP 1 380 820 A1

(12) **DEMANDE DE BREVET EUROPEEN**

(43) Date de publication:
**14.01.2004 Bulletin 2004/03**

(51) Int Cl.⁷: **G01J 3/46**

(21) Numéro de dépôt: **03101986.2**

(22) Date de dépôt: **03.07.2003**

(84) Etats contractants désignés:
**AT BE BG CH CY CZ DE DK EE ES FI FR GB GR HU IE IT LI LU MC NL PT RO SE SI SK TR**
Etats d'extension désignés:
**AL LT LV MK**

(30) Priorité: **11.07.2002 FR 0208762**

(71) Demandeur: **L'OREAL S.A.**
**75008 Paris Cedex (FR)**

(72) Inventeurs:
- **Audousset, Marie-Pascale**
**92600, Asnieres (FR)**
- **Millequant, Jean-Marie**
**94100, Saint Maur des Fosses (FR)**

(74) Mandataire: **Kohn, Philippe et al**
**Cabinet Philippe Kohn,**
**30, rue Hoche**
**93500 Pantin (FR)**

(54) **Procédé de détermination de la composition d'un produit de coloration**

(57) L'invention propose un procédé de détermination de la composition d'un produit de coloration de fibres kératiniques comportant :

- une étape de saisie d'au moins une donnée représentative d'une coloration cible à atteindre ; et
- une étape de détermination, à partir d'une base de données, d'au moins un produit de coloration qui permet d'obtenir la coloration cible ou une coloration théorique qui s'écarte au plus de la coloration cible d'une valeur théorique prédéterminée, ou d'un nombre prédéterminé de produits qui permettent d'obtenir des colorations théoriques les plus proches de la coloration cible.

Selon l'invention, le procédé comporte une étape d'affichage de la composition de chaque produit déterminé, identifiant le ou les éléments colorants nécessaires à leur réalisation, ainsi que les proportions respectives desdits éléments nécessaires

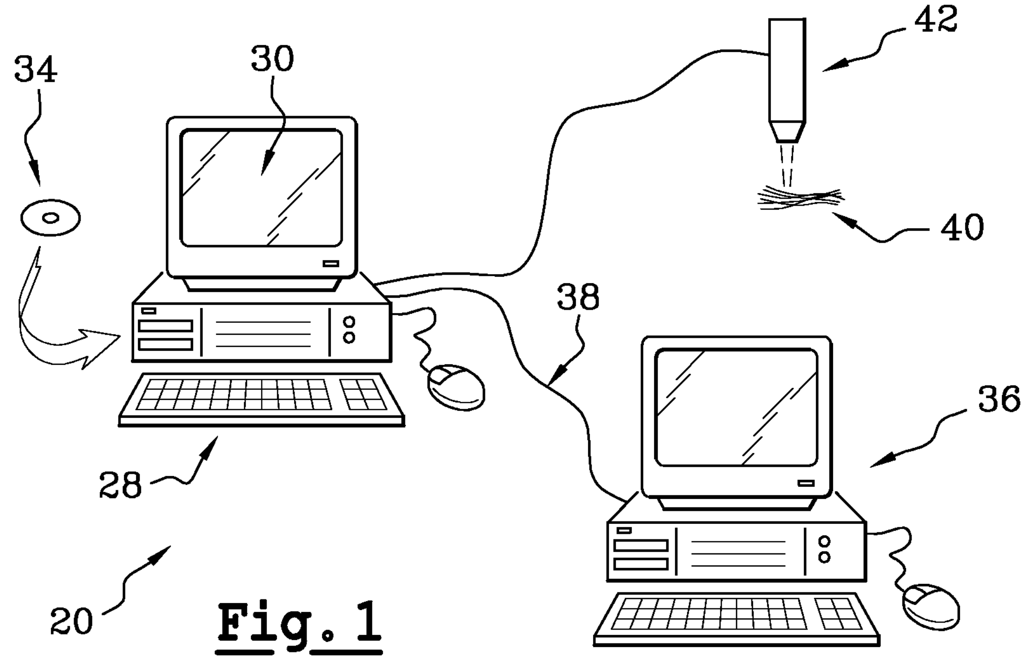


**Fig. 1**



Printed by Jouve, 75001 PARIS (FR)

## Description

L'invention propose un procédé de détermination de la composition d'un produit de coloration des fibres kératiniques.

L'invention propose plus particulièrement un procédé de détermination de la composition d'un produit de coloration de fibres kératiniques comportant une étape de saisie d'au moins une donnée représentative d'une coloration cible à atteindre et une étape de détermination, à partir d'une base de données, d'au moins un produit qui permet d'obtenir la coloration cible ou une coloration théorique qui s'écarte au plus de la coloration cible d'une valeur théorique prédéterminée, ou d'un nombre prédéterminé de produits qui permettent d'obtenir les colorations théoriques les plus proches de la coloration cible.

La coloration capillaire est une partie de la cosmétique actuelle qui permet à une personne non seulement de masquer ses cheveux blancs, mais aussi de modifier la couleur de sa chevelure.

Il existe plusieurs types de coloration des cheveux. Il y a la coloration temporaire ou "fugace" dont les effets s'estompent au moindre shampooing, la coloration semi-permanente ou directe dont les effets s'estompent très lentement après plusieurs shampoings, et la coloration permanente qui permet d'avoir une modification de la pigmentation des cheveux.

Les produits de coloration permettent d'obtenir trois types de résultats :

- ajouter à la couleur naturelle de la chevelure des reflets divers ;
- colorer les cheveux blancs dans les tons naturels pour ramener l'ensemble de la chevelure dans sa nuance originelle, ou dans une nuance plus claire ou plus foncée ; et
- « déjaunir » les cheveux gris ou blancs en leur apportant une tonalité grise, le type de produit utilisé étant dit de "reflets gris".

Dans la suite de la présente demande, par "élément colorant", on entend une entité à un ou plusieurs constituants, apte à elle seule à colorer. Une telle entité est constituée notamment :

i) d'une base seule ;
ii) d'une base et d'un coupleur ; ou
iii) d'un colorant direct.

Ainsi, les produits de coloration directe comportent, en tant qu'élément colorant, un ou plusieurs colorants, en fonction du résultat souhaité.

Les produits de coloration d'oxydation comportent, en tant qu'élément colorant, au moins une base et généralement au moins un coupleur, et éventuellement un ou plusieurs colorants directs.

Les éléments colorants sont introduits dans un support, qui est par exemple une crème ou un gel, et qui facilite l'application du produit de coloration sur la chevelure.

Chaque mélange de base avec un coupleur donne naissance par oxydation, en présence d'un agent oxydant tel que par exemple le peroxyde d'hydrogène, et par polymérisation, à un pigment qui se fixe dans le cheveu.

Le nombre existant de bases et de coupleurs est limité dans la pratique. Cependant, les divers mélanges possibles de ces bases et coupleurs augmentent considérablement le nombre total de compositions colorantes.

La diversité des couleurs naturelles de cheveux qui vont du noir au blond très clair, ainsi que la diversité des goûts des clients en ce qui concerne les couleurs et les reflets désirés, complexifient la tache du coiffeur dans le choix du produit de coloration adéquat.

Il est alors difficile, même pour un coiffeur expérimenté de réaliser même par mélange, le produit colorant permettant d'obtenir exactement la coloration désirée.

Les fabricants proposent des produits de coloration fabriqués à l'avance, et des exemples de colorations obtenues avec ce produit sont affichés sur l'emballage du produit ou sur un nuancier. Cependant, du fait de la diversité des chevelures, notamment de leurs couleurs qui varient du blond au brun et qui comportent un certain pourcentage de cheveux blancs, un même produit de coloration ne permet pas d'obtenir le même résultat, et la coloration finale obtenue n'est pas toujours celle attendue.

Selon les enseignements du document US-A-6.330.341, une base de données donnant, pour chaque produit de coloration existant dans le commerce, la coloration finale obtenue en fonction de la couleur initiale des cheveux est constituée. Cette base de données regroupe un grand nombre de produits existant dans le commerce.

Ce document décrit aussi un procédé qui permet de déterminer le ou les produits de coloration commerciaux permettant d'obtenir la coloration voulue.

Un tel procédé nécessite que la personne qui le met en oeuvre dispose de la totalité des produits enregistrés dans la base de données, ce qui n'est pas envisageable pour un salon de coiffure, car cela imposerait un trop grand stock de produits. De plus, ce procédé est limité aux produits existant dans le commerce.

Le nombre d'éléments colorants qui entrent dans la composition des produits de coloration, que ce soit pour une coloration directe ou pour une coloration d'oxydation, est moins important que le nombre de produits de coloration pouvant être commercialisés.

Il est donc préférable, pour une petite structure comme un salon de coiffure, de disposer des éléments colorants sous forme individualisée, et de réaliser le produit de coloration au fur et à mesure de la demande de ses clients en mélangeant les éléments nécessaires

pour obtenir la coloration voulue.

Cependant, comme on l'a dit plus haut, il n'est pas facile pour un coiffeur de formuler exactement le produit de coloration permettant d'obtenir la coloration voulue.

L'invention propose un procédé permettant d'élaborer le produit de coloration nécessaire à partir des éléments colorants de base.

L'invention propose donc un procédé d'élaboration d'un produit de coloration du type décrit précédemment, caractérisé en ce qu'il comporte une étape d'affichage de la composition de chaque produit déterminé, citant les éléments colorants nécessaires à la réalisation du produit, ainsi que les proportions de chaque élément nécessaire.

Selon d'autres caractéristiques de l'invention :

- l'étape d'affichage consiste aussi à afficher, pour chaque produit déterminé, au moins une valeur représentative de l'écart entre la coloration théorique obtenue par le produit et la coloration cible ;
- l'étape d'affichage consiste aussi à afficher, pour chaque produit déterminé, un élément graphique coloré dont la coloration est la coloration théorique obtenue par le produit ;
- l'étape d'affichage consiste à afficher les produits déterminés en les ordonnant par ordre de proximité entre la coloration théorique et la coloration cible ;
- l'étape de détermination consiste à sélectionner un ou plusieurs produits présents dans la base de données et/ou à déterminer la composition d'un ou plusieurs produits par calcul numérique ;
- l'élément colorant peut être constitué d'une base d'oxydation et éventuellement d'un coupleur d'oxydation ;
- l'élément colorant peut être constitué d'un colorant direct ;
- le procédé peut comporter une étape de saisie d'au moins une caractéristique des cheveux à colorer de préférence préalablement à l'étape de saisie de la couleur cible ;
- ladite caractéristique peut être une donnée représentative de la couleur réelle des cheveux à colorer ;
- le procédé peut comporter une étape d'identification d'un ensemble réduit d'éléments colorants choisis parmi tous les éléments colorants contenus dans la base de données, l'étape de détermination consistant à déterminer des produits de colorations formés d'un ou plusieurs éléments colorants dudit ensemble réduit.

L'invention propose aussi un procédé de préparation d'un produit de coloration, consistant à :

i) au moyen du procédé de détermination décrit précédemment, déterminer la proportion d'un ou plusieurs éléments colorants entrant dans le produit de coloration à préparer ; et

ii) préparer ledit produit de coloration en y incorporant le ou les éléments colorant déterminé lors de l'étape i) selon les proportions déterminé lors de ladite étape i).

L'invention propose aussi une base de données susceptible d'être utilisée dans la mise en oeuvre d'un des procédés décrits précédemment, caractérisée en ce qu'elle comporte :

a) des premières données pour identifier une pluralité d'éléments colorants,

b) des deuxièmes données comportant :

i) la concentration du ou des éléments colorants, choisis parmi ladite pluralité d'éléments colorants, et entrant dans la préparation d'une pluralité de produits de coloration ; et

ii) pour chacun desdits produits de coloration, la couleur obtenue lorsqu'ils sont appliqués selon des conditions prédéfinies.

Les conditions prédéfinies sont notamment des conditions relatives au pourcentage de mélange avec un oxydant de force variable, le type de fibres sur lesquelles le produit a été appliqué, le taux de cheveux blancs et/ou sensibilisés, la température, et le temps de pose. Ces conditions prédéfinies peuvent ou non faire partie des informations affichées.

Selon d'autres caractéristiques de l'invention, la base de données comporte des troisièmes données représentatives de caractéristiques économiques et/ou réglementaires d'au moins un desdits produits de coloration.

L'invention propose aussi un procédé de constitution d'une base de données décrite précédemment, caractérisé en ce qu'il comporte :

i) une étape d'enregistrement des premières données, et

ii) une étape d'enregistrement des deuxièmes données associées.

Selon d'autres caractéristiques de l'invention, le procédé de constitution de la base de données comporte une étape de mise à jour de la base de données en enregistrant les deuxièmes données relatives à au moins un nouveau produit de coloration.

L'étape de mise à jour peut comporter l'enregistrement d'au moins une nouvelle première donnée.

L'invention propose aussi un agencement pour la détermination de la composition d'un produit de coloration de fibres kératiniques, en mettant en oeuvre un procédé décrit précédemment, caractérisé en ce qu'il comporte :

i) des moyens de saisie d'au moins une donnée représentative d'une coloration cible ;

ii) des moyens de calcul pour déterminer, à partir d'une base de données, au moins un produit qui permet d'obtenir la coloration cible ou une coloration théorique qui s'écarte au plus de la coloration cible d'une valeur théorique prédéterminée, ou d'un nombre prédéterminé de produits qui permettent d'obtenir des colorations théoriques les plus proches de la coloration cible, et

iii) des moyens d'affichage de la composition de chaque produit déterminé, identifiant les éléments colorants nécessaires à leur élaboration, ainsi que leurs proportions respectives.

Selon d'autres caractéristiques de l'invention :

- la base de données, et éventuellement les moyens de calcul, sont délocalisés relativement aux moyens de saisie et aux moyens d'affichage, les données entre au moins certains constituants de l'agencement pouvant transiter par un réseau informatique de type Internet ;
- les moyens de saisie de la coloration cible peuvent comporter un colorimètre électronique ou un spectrocolorimètre électronique.

D'autres caractéristiques et avantages de l'invention apparaîtront à la lecture de la description détaillée qui suit pour la compréhension de laquelle on se reportera aux figures annexées parmi lesquelles :

- la figure 1 est une représentation schématique d'un agencement conforme à l'invention ;
- la figure 2 est une représentation schématique d'une base de donnée dans laquelle les premières, deuxièmes, troisièmes et quatrièmes données sont réparties en colonnes, et dans laquelle chaque ligne détermine la formulation d'un produit de coloration ;
- la figure 3 est un graphe représentatif de la valeur du coefficient K/S en fonction de la concentration du produit de coloration , pour un produit de coloration donné et pour une valeur de longueur d'onde donnée ;
- la figure 4 est un autre graphe représentatif de la valeur du coefficient K/S en fonction de la longueur d'onde, pour un même produit de coloration et dans lequel chaque courbe correspond à une concentration donnée du produit de coloration.

Un produit de coloration peut comprendre une ou plusieurs bases d'oxydation choisies parmi les paraphénylènediamines, les bis-phénylalkylènediamines, les para-aminophénols, les ortho-aminophénols, les bases hétérocycliques et leurs sels d'addition.

Parmi les paraphénylènediamines, on peut citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl) amino 2-méthyl aniline, la 4-N,N-bis-(β-hydroxyéthyl) amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, la 4-aminophénylpyrrolidine, la 2-thiényl paraphénylènediamine, le 2-β hydroxyéthylamino 5-amino toluène, la 3-hydroxy 1-(4'-aminophényl) pyrrolidine et leurs sels d'addition avec un acide.

Parmi les paraphénylènediamines citées ci-dessus, la paraphénylènediamine, la paratoluylènediamine, la 2-isopropyl paraphénylènediamine, la 2-β-hydroxyéthyl paraphénylènediamine , la 2-β-hydroxyéthyloxy paraphénylène-diamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-amino phényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition avec un acide.

Parmi les para-aminophénols, on peut citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido

2-amino phénol, et leurs sels d'addition avec un acide.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl) amino 3-amino 6-méthoxy pyridine, la 3,4-diamino pyridine, et leurs sels d'addition avec un acide.

D'autres bases d'oxydation pyridiniques sont les bases d'oxydation 3-amino pyrazolo-[1,5-a]-pyridines ou leurs sels d'addition décrits par exemple dans la demande de brevet FR 2801308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine; l'acide 3-amino-pyrazolo[1,5-a]pyridin-2-carboxylique ; la 2-méthoxypyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol; le (3-amino-pyrazolo[1,5-a]pyridine-2-yl)-méthanol ; la 3,6-diamino-pyrazolo[1,5-a]pyridine ; la 3,4-diamino-pyrazolo[1,5-a] pyridine ; la pyrazolo[1,5-a]pyridine-3,7-diamine; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl) -(2-hydroxyéthyl)-amino]-éthanol ; la 3-aminopyrazolo [1,5-a]pyridine-5-ol ; 3-amino-pyrazolo[1,5-a]pyridine-4-ol ; la 3-amino-pyrazolo[1,5-a]pyridine-6-ol ; la 3-aminopyrazolo[1,5-a]pyridine-7-ol ;

ainsi que leurs sels d'addition avec un acide ou avec une base.

Parmi les dérivés pyrimidiniques, on peut citer les composés décrits par exemple dans les brevets DE 2359399 ; JP 88-169571 ; JP 05-63124 ; EP 0770375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2750048 et parmi lesquels on peut citer la pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo [1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine et leurs sels d'addition avec un acide et leurs formes tautomères, lorsqu'il existe un équilibre tautomérique.

Parmi les dérivés pyrazoliques, on peut citer les composés décrits dans les brevets DE 3843892, DE 4133957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

La ou les bases d'oxydation présentes dans le produit de coloration sont en général présentent chacune en quantité comprise entre 0,001 à 10 % en poids environ du poids total de la composition tinctoriale, de préférence entre 0,005 et 6 %.

Le produit de coloration peut aussi contenir un ou plusieurs coupleurs conventionnellement utilisés pour la teinture de fibres kératiniques. Parmi ces coupleurs, on peut notamment citer les métaphénylènediamines, les méta-aminophénols, les métadiphénols, les coupleurs naphtaléniques, les coupleurs hétérocycliques et leur sels d'addition.

A titre d'exemple, on peut citer le 2-méthyl 5-aminophénol, le 5-N-(β-hydroxyéthyl)amino 2-méthyl phénol, le 6-chloro-2-méthyl-5-aminophénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition avec un acide.

Dans le produit de coloration, le ou les coupleurs sont chacun généralement présents en quantité comprise entre 0,001 et 10 % en poids environ du poids total de la composition tinctoriale, de préférence entre 0,005 et 6 %.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates et les sels d'addition avec une base telles que la soude, la potasse, l'ammoniaque, les amines ou les alcanolamines.

Les colorants directs pouvant entrer dans la composition du produit de coloration sont choisis de préférence parmi les colorants directs nitrés benzéniques neutres, acides ou cationiques, les colorants directs azoïques neutres acides ou cationiques, les colorants directs quinoniques et en particulier anthraquinoniques neutres, acides ou cationiques, les colorants directs aziniques, les colorants directs triarylméthaniques, les colorants directs indoaminiques et les colorants directs naturels.

Parmi les colorants directs benzéniques pouvant entrer dans la composition du produit de coloration, on peut citer de manière non limitative les composés suivants:

- 1,4-diamino-2-nitrobenzène
- 1-amino-2 nitro-4-β- hydroxyéthylaminobenzène
- 1-amino-2 nitro-4-bis(β-hydroxyéthyl)-aminobenzène
- 1,4-Bis(β-hydroxyéthylamino)-2-nitrobenzène
- 1-β-hydroxyéthylamino-2-nitro-4-bis-(β-hydroxyéthylamino)-benzène
- 1-β-hydroxyéthylamino-2-nitro-4-aminobenzène
- 1-β-hydroxyéthylamino-2-nitro-4-(éthyl)(β-hydroxyéthyl)-aminobenzène
- 1-amino-3-méthyl-4-β-hydroxyéthylamino-β-nitrobenzène
- 1-amino-2-nitro-4-β-hydroxyéthylamino-5-chlorobenzène
- 1,2-Diamino-4-nitrobenzène
- 1-amino-2-β-hydroxyéthylamino-5-nitrobenzène
- 1,2-Bis-(β-hydroxyéthylamino)-4-nitrobenzène
- 1-amino-2-tris-(hydroxyméthyl)-méthylamino-5-nitrobenzène
- 1-Hydroxy-2-amino-5-nitrobenzène
- 1-Hydroxy-2-amino-4-nitrobenzène
- 1-Hydroxy-3-nitro-4-aminobenzène
- 1-Hydroxy-2-amino-4,6-dinitrobenzène
- 1-β-hydroxyéthyloxy-2-β-hydroxyéthylamino-5-nitrobenzène
- 1-Méthoxy-2-β-hydroxyéthylamino-5-nitrobenzène
- 1-β-hydroxyéthyloxy-3-méthylamino-4-nitrobenzène
- 1-β,γ-dihydroxypropyloxy-3-méthylamino-4-nitrobenzène
- 1-β-hydroxyéthylamino-4-β,γ-dihydroxypropyloxy-2-nitrobenzène
- 1-β,γ-dihydroxypropylamino-4-trifluorométhyl-2-nitrobenzène
- 1-β-hydroxyéthylamino-4-trifluorométhyl-2-nitrobenzène
- 1-β-hydroxyéthylamino-3-méthyl-2-nitrobenzène
- 1-β-aminoéthylamino-5-méthoxy-2-nitrobenzène
- 1-Hydroxy-2-chloro-6-éthylamino-4-nitrobenzène
- 1-Hydroxy-2-chloro-6-amino-4-nitrobenzène
- 1-Hydroxy-6-bis-(β-hydroxyéthyl)-amino-3-nitrobenzène
- 1-β-hydroxyéthylamino-2-nitrobenzène
- 1-Hydroxy-4-β-hydroxyéthylamino-3-nitrobenzène.

Parmi les colorants directs azoïques pouvant entrer dans la composition du produit de coloration, on peut citer les colorants azoiques cationiques décrits dans les demandes de brevets WO 95/15144, WO-95/01772 et EP-714954 dont le contenu fait partie intégrante de l'invention.

Parmi ces composés on peut tout particulièrement citer les colorants suivants:

- chlorure de 1,3-diméthyl-2-[[4-(diméthylamino) phényl]azo] -1H-Imidazolium,
- chlorure de 1,3-diméthyl-2-[(4-aminophényl)azo]-1H-Imidazolium,
- méthylsulfate de 1-méthyl-4-[(méthylphénylhydrazono)mét hyl]-pyridinium.

On peut aussi citer parmi les colorants directs azoïques les colorants suivants, décrits dans le COLOUR INDEX INTERNATIONAL 3e édition :

- Disperse Red 17
- Acid Yellow 9
- Acid Black 1
- Basic Red 22
- Basic Red 76
- Basic Yellow 57
- Basic Brown 16
- Acid Yellow 36
- Acid Orange 7
- Acid Red 33
- Acid Red 35
- Basic Brown 17
- Acid Yellow 23
- Acid Orange 24
- Disperse Black 9.

On peut aussi citer le 1-(4'-aminodiphénylazo)-2-méthyl-4bis-(β-hydroxyéthyl) aminobenzène et l'acide 4-hydroxy-3-(2-méthoxyphénylazo)-1-naphtalène sulfonique.

Parmi les colorants directs quinoniques on peut citer les colorants suivants :

- Disperse Red 15
- Solvent Violet 13
- Acid Violet 43
- Disperse Violet 1
- Disperse Violet 4
- Disperse Blue 1
- Disperse Violet 8
- Disperse Blue 3
- Disperse Red 11
- Acid Blue 62
- Disperse Blue 7
- Basic Blue 22
- Disperse Violet 15
- Basic Blue 99

ainsi que les composés suivants :

- 1-N-méthylmorpholiniumpropylamino-4-hydroxyanthraquinone
- 1-Aminopropylamino-4-méthylaminoanthraquinone
- 1-Aminopropylaminoanthraquinone
- 5-β-hydroxyéthyl-1,4-diaminoanthraquinone
- 2-Aminoéthylaminoanthraquinone
- 1,4-Bis-(β,γ-dihydroxypropylamino)-anthraquinone.

Parmi les colorants aziniques on peut citer les composés suivants :

- Basic Blue 17
- Basic Red 2.

Parmi les colorants triarylméthaniques pouvant entrer dans la composition du produit de coloration, on peut citer les composés suivants :

- Basic Green 1
- Acid blue 9
- Basic Violet 3
- Basic Violet 14
- Basic Blue 7
- Acid Violet 49
- Basic Blue 26
- Acid Blue 7

Parmi les colorants indoaminiques pouvant entrer dans la composition du produit de coloration, on peut citer les composés suivants :

- 2-β-hydroxyéthlyamino-5-[bis-(β-4'-hydroxyéthyl) amino]anilino-1,4-benzoquinone
- 2-β-hydroxyéthylamino-5-(2'-méthoxy-4'-amino) anilino-1,4-benzoquinone
- 3-N(2'-Chloro-4'-hydroxy)phényl-acétylamino-6-méthoxy-1,4-benzoquinone imine
- 3-N(3'-Chloro-4'-méthylamino)phényl-uréido-6-méthyl-1,4-benzoquinone imine
- 3-[4'-N-(Ethyl,carbamylméthyl)-amino]-phényl-uréido-6-méthyl-1,4-benzoquinone imine

Parmi les colorants directs naturels pouvant entrer dans la composition du produit de coloration, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine. On peut aussi utiliser les extraits ou décoctions contenant ces colorants naturels et notamment les cataplasmes ou extraits à base de henné.

Le ou les colorants directs représentent de préférence de 0,001 à 20% en poids environ du poids total du produit de coloration prêt à l'emploi et encore plus préférentiellement de 0,005 à 10% en poids environ.

Le milieu approprié pour la teinture appelé aussi support de teinture est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C1-C4, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants sont, de préférence, présents dans des proportions de préférence comprises entre 1 et 40 % en poids environ par rapport au poids total de la composition tinctoriale, et encore plus préférentiellement entre 5 et 30 % en poids environ.

Le produit de coloration peut aussi renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Les adjuvants ci dessus sont en général présents en quantité comprise pour chacun d'eux entre

0,01 et 20 % en poids par rapport au poids du produit de coloration.

Le pH du produit de coloration est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule suivante :

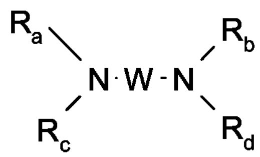

dans laquelle W est un reste propylène éventuellement substitué par un groupement hydroxyle ou un radical alkyle en C1-C4 ; Ra, Rb, Rc et Rd, identiques ou différents, représentent un atome d'hydrogène, un radical alkyle en C1-C4 ou hydroxyalkyle en C1-C4.

Le produit de coloration peut se présenter sous des formes diverses, telles que sous forme de liquides, de crèmes, de gels, ou sous toute autre forme appropriée pour réaliser une teinture des fibres kératiniques, et notamment des cheveux humains.

Les instruments modernes mesurent la quantité de lumière qui est réfléchie par un échantillon coloré à diverses longueurs d'onde, pour établir les caractéristiques spectrales de l'objet illuminé.

Mis à part le noir qui ne réfléchit aucune lumière, et le blanc qui réfléchit toute la lumière, toutes les couleurs réfléchissent la lumière seulement dans certaines régions spécifiques du spectre visible. Dans ces cas, elles ont des formes de courbes spectrales caractéristiques qui forment des "cartes d'identité" des couleurs, deux couleurs n'étant identiques que si leurs spectres sont superposables.

Pour faire une description objective et systématique des couleurs, et contrairement à la perception visuelle qui est subjective car elle dépend notamment de l'observateur et des conditions d'observation, on a besoin d'un système colorimétrique normalisé. Pour la représentation d'un point de couleur dans l'espace et pour le calcul des écarts de couleurs, le système colorimétrique CIELAB mis en place par la Commission Internationale de l'Eclairage (CIE) est le plus utilisé actuellement.

Ce système utilise trois valeurs L*, a* et b*, dans lesquelles la valeur L* exprime la clarté, et les valeurs a* et b* expriment la teinte et la saturation d'une couleur.

Ainsi, L* varie du noir au blanc, a* varie du vert au rouge en passant par le gris, et b* exprime la variation du bleu au jaune en passant aussi par le gris.

L'écart entre deux couleurs peut s'exprimer par la relation :

$$\Delta E^* = \sqrt{(\Delta L^*)^2 + (\Delta a^*)^2 + (\Delta b^*)^2}$$

Chaque élément colorant possède une courbe d'étalonnage qui est la courbe spectrale de la coloration qu'il permet d'obtenir sur un type de chevelure donné et en fonction de sa concentration.

L'invention propose une base de données 10 permettant de déterminer, pour un élément colorant ou pour un mélange d'éléments colorants donnés, la coloration obtenue.

On peut par exemple constituer la base de données 10 en enregistrant les valeurs de L*, a* et b* pour chaque composition.

On peut aussi constituer la base de données 10 à partir de la coefficients K/S déterminés à une ou plusieurs longueurs d'onde.

Le coefficient K/S est déterminé à partir de la réflectance R de la couleur considérée par la relation $K/S = \frac{(1 - R^2)}{2R}$, la réflectance R étant le rapport entre l'énergie lumineuse réémise et l'énergie incidente.

On a représenté aux figures 3 et 4 des exemples de courbes d'étalonnage d'un mélange binaire d'une base d'oxydation, la paraphénylènediamine, et d'un coupleur d'oxydation, la résorcine.

La figure 3 est une courbe représentant le coefficient K/S en fonction de la concentration de l'élément colorant, et pour une longueur d'onde donnée.

La courbe représentant le coefficient K/S en fonction de la concentration de l'élément colorant n'est pas rectiligne, ce qui montre que le coefficient K/S n'est pas proportionnel à la concentration.

Ainsi, il n'est pas possible de déterminer la coloration obtenue par un élément colorant à une concentration données uniquement par la multiplication de la valeur du coefficient K/S par un coefficient de proportionnalité.

Il en est de même à la figure 4, dans laquelle la courbe représentative de K/S en fonction de la longueur d'onde, n'est également pas rectiligne.

Ainsi, pour chaque composition de produit de coloration, il est nécessaire d'enregistrer plusieurs valeurs du coefficient K/S pour différentes concentration et pour différentes longueurs d'onde.

C'est pourquoi, comme on l'a représenté à la figure 2, la base de données 10 comporte un ensemble 12 de données 14 qui sont des valeurs représentatives de la couleur réelle obtenue par chaque produit de coloration.

La base de données 10 comporte aussi des données 16 qui permettent d'identifier un ensemble d'éléments colorants, aptes à entrer dans les compositions des produits de colorations que l'on veut déterminer.

Chacune de ces données 16 permet d'identifier un seul élément colorant, et elle peut consister soit en un nom chimique de l'élément colorant, soit en un code, ou bien consister en une structure chimique de l'élément colorant.

Selon une variante de réalisation de l'invention, la base de données 10 comporte un autre ensemble 18 de données 20 relatives aux conditions d'application de chaque produit de coloration.

Enfin, la base de données 10 peut comporter un ensemble 22 de données 24 qui sont représentatives de caractéristiques économiques ou réglementaires relatives aux produits de coloration. Ces caractéristiques peuvent être par exemple le prix de chaque produit de coloration, des données toxicologiques, ou des données relatives aux droits de propriété industrielle.

Ainsi, comme on peut le voir à la figure 2, la base de données 10 comporte des premières données 16 qui sont les données d'identification des éléments colorants, des deuxièmes données 14 qui permettent de définir la coloration obtenue par chaque produit de coloration, des troisièmes données 24 représentatives de caractéristiques économiques ou réglementaires du produit de coloration, et des quatrièmes données 20 qui permettent de définir les conditions d'application des produits de coloration.

Pour chaque produit de coloration, la base de données 10 associe une valeur à chacune des premières données 16, cette valeur étant la proportion de l'élément colorant identifié par la première donnée 16 associée dans le produit de coloration. Si un élément colorant n'entre pas dans la composition du produit de coloration donné, la valeur associée à cet élément colorant est nulle, et selon une variante de réalisation de l'invention, aucune valeur est affichée lorsque l'élément colorant n'entre pas dans la composition du produit de coloration.

Aussi, pour chaque produit de coloration, la base de données 10 associe une valeur à chacune des deuxièmes données 14, cette valeur étant ici la valeur du coefficient K/S pour une valeur de longueur d'onde, la base de données 10 associe aussi une valeur à chacune des troisièmes et quatrièmes données 20, 24, ces valeurs pouvant être numériques ou bien alphanumériques.

L'invention propose aussi un procédé pour l'élaboration d'une telle base de données 10.

Ce procédé comporte une première étape d'enregistrement des premières données, c'est-à-dire la désignation de chaque élément colorant pouvant entrer dans la composition d'un produit de coloration, et une deuxième étape d'enregistrement d'une pluralité de produits de coloration.

La deuxième étape d'enregistrement de la pluralité de produits de coloration consiste à enregistrer, pour chaque produit de coloration, la proportion de chaque élément colorant qui entre dans la composition du produit de coloration, et à associer cette proportion à la première donnée 16 identifiant l'élément colorant.

Le procédé de constitution de la base de données 10 nécessite de réaliser des essais de chaque produit de coloration sur des échantillons de fibres, en fonction des divers ensembles de données relatives aux produits de coloration.

La coloration obtenue par chaque produit de coloration sur un échantillon de fibres donné est alors mesurée, par exemple par un spectrocolorimètre électronique. Ensuite, les valeurs du coefficient K/S pour différentes valeurs de longueur d'onde sont déterminées puis elles sont enregistrées dans la base de données 10.

L'élaboration de la base de données 10 est relativement longue car elle nécessite la réalisation de nombreux essais.

C'est pourquoi le procédé de constitution de la base de données 10 est réalisée en plusieurs étapes, et il comporte une première étape d'enregistrement des produits de coloration dont la coloration finale est connue, et il comporte au moins une étape de mise à jour de la base de données 10 qui consiste à enregistrer les produits de coloration dont la coloration finale a été déterminée depuis la première étape d'enregistrement ou depuis l'étape de mise à jour précédente.

Une fois la base de données 10 constituée, elle est mise à la disposition de l'utilisateur qui est le professionnel qui va élaborer les produits de coloration. Il peut être un coiffeur opérant dans un salon de coiffure ou bien un fabricant de produits de colorations.

L'utilisateur a à sa disposition un dispositif électronique 26 tel que représenté à la figure 1 qui comporte notamment des moyens de saisie 28 de la coloration cible, des moyens d'affichage 30 et des moyens d'interrogation de la base de données 10. Le dispositif électronique 26 peut être par exemple un ordinateur, dans lequel la base de données 10 est enregistrée, dans un composant interne, comme par exemple un disque dur, ou bien sur un composant amovible, comme par exemple sur un CD ROM 34.

L'ordinateur peut aussi être relié à un deuxième ordinateur 36 par tout moyen connu, comme par exemple un réseau informatique 38 qui peut être interne, dans quel cas on parle d'un "intranet", ou par un réseau informatique externe comme par exemple l'Internet.

Une étape de mise à jour s'effectue en envoyant à l'utilisateur un programme qui est enregistré

sur tout support conventionnel comme par exemple un CD-ROM, l'utilisateur devant alors exécuter le programme pour que la mise à jour s'effectue.

Selon un autre mode de réalisation de l'invention, lorsque l'ordinateur qui est à la disposition de l'utilisateur est relié à un deuxième ordinateur central, la base de données 10 est uniquement enregistrée dans ce deuxième ordinateur.

Ainsi, lors de l'étape de sélection, l'ordinateur interroge la base de données 10 qui est enregistrée dans le deuxième ordinateur.

Ceci permet de simplifier les mises à jour successives de la base de donnée car il n'y a qu'une seule base de données 10 à modifier à la place de la quantité des bases de données que peuvent former l'ensemble des utilisateurs.

De plus, un fois la mise à jour de la base de données 10 de l'ordinateur central effectuée, l'ensemble des utilisateurs qui appliquent le procédé ont à leur disposition les nouvelles compositions colorantes, sans avoir à effectuer une tache de mise à jour qui peut être compliquée.

L'invention propose aussi un procédé pour la détermination de la composition d'un produit de coloration qui permet d'obtenir une coloration cible voulue par une personne, ou bien une coloration proche de la coloration cible.

Pour cela, une première étape du procédé conforme à l'invention, consiste à saisir sous forme électronique la coloration cible.

Cette étape de saisie peut s'effectuer à partir d'un nuancier qui se présente sous forme informatique. Dans ce cas l'ordinateur 26 mettant en oeuvre le procédé de détermination comporte un logiciel d'aide à la sélection des colorations. Selon une variante de réalisation de l'invention, l'étape de saisie s'effectue à partir d'un nuancier qui se présente sous la forme d'un catalogue « matériel» par exemple en papier, et qui comporte un code unique associé à chacune des colorations qu'il regroupe.

Lorsque la coloration voulue n'est pas présente dans le nuancier, mais qu'elle est présente sur un autre support, comme par exemple une mèche 40 de la chevelure d'une tierce personne ou un catalogue de mèches colorées, l'étape de saisie de la coloration cible consiste à déterminer électroniquement la courbe spectrale de la coloration cible.

Pour cela, les moyens de saisie 28 du dispositif électronique consistent par exemple en un colorimètre électronique 42 qui sont reliés à l'ordinateur 26 de l'utilisateur.

À titre d'exemple non limitatif de colorimètres , ou spectrocolorimètres, on cite le Spectraflash SF600, le Spectraflash SF300, le Microflash 200d, ou les Mercury 1000 et 2000 commercialisés par la société DATACOLOR, et les CM 1000, 2000 ou 3000, commercialisés par la société MINOLTA.

Lorsque certaines des troisièmes données 24 de la base de données 10 sont relatives à la coloration initiale des cheveux à colorer, l'étape de saisie comporte aussi une étape d'analyse de la chevelure à colorer qui s'effectue de la même manière que l'étape de saisie de la coloration cible, c'est-à-dire soit directement sur l'ordinateur 26, à partir d'un nuancier ou bien par l'intermédiaire du spectrocolorimètre 42 qui est à la disposition de l'utilisateur.

Cette étape peut permettre soit de sélectionner la bonne base de données ou le bon sous-ensemble de la base de données, soit d'introduire un facteur correctif pour les calculs ultérieurs

Ensuite, selon une deuxième étape du procédé, l'ordinateur 26 détermine par calcul une ou plusieurs compositions de produits de coloration qui permettent d'obtenir la coloration cible ou une coloration théorique proche de la coloration cible.

Pour cela, l'ordinateur 26 interroge la base de données 10 et il calcule l'écart $\Delta E^*$ entre la coloration théorique obtenue par chacun des produits de coloration qui sont enregistrés dans la base de données 10, et la coloration cible.

L'ordinateur 26 détermine aussi des composition de produits de coloration qui ne sont pas enregistrées dans la base de données 10 et il calcule l'écart $\Delta E^*$ entre la coloration théorique obtenue par chacune des compositions déterminées et la coloration cible.

Enfin, l'ordinateur 26 sélectionne les produits de coloration qui permettent d'obtenir la coloration cible.

Lorsque aucun produit de coloration ne permet d'obtenir une coloration théorique identique à la coloration cible, l'ordinateur 26 sélectionne les produits de coloration qui permettent d'obtenir une coloration théorique proche de la coloration cible.

Une coloration étant considérée comme proche de la coloration cible lorsque l'écart $\Delta E^*$ est inférieur ou égal à une valeur prédéterminée. À titre d'exemple non limitatif, on prendra pour valeur prédéterminée de l'écart $\Delta E^*$ la valeur minimale pour laquelle l'écart entre deux colorations est perceptible par l'oeil humain qui est égale à 2.

Selon une variante de réalisation de l'invention, l'ordinateur 26 sélectionne un nombre prédéterminé de produits de coloration qui permettent d'obtenir des colorations théoriques les plus proches de la coloration cible.

Cette étape de détermination est réalisée par l'intermédiaire d'un logiciel de formulation, qui est par exemple le logiciel Datamatch ou DCI Match, commercialisés par la société DATACOLOR, Isomatch, commercialisé par la société SPC, Prisma ou Spectramatch commercialisés par la société MINOLTA, QuicInkplus commercialisé par la société X-RITE, "Ink Formulation and Mixing" et "Propalette Textile" commercialisés par la société GRETAGMACBETH.

A l'issue de l'étape de détermination, le procédé comporte une étape d'affichage des résultats de l'étape de détermination qui consiste à afficher, sur

l'écran 30 de l'ordinateur 26 ou bien en les imprimant sur du papier, la composition de chacun des produits de coloration déterminés.

L'étape d'affichage consiste à afficher les premières données 16 identifiant les éléments colorants qui entre dans la composition du produit de coloration, ainsi que la proportion de chacun des éléments colorants sous la forme d'une liste.

Pour faciliter le choix du produit de coloration qui sera élaborée, et selon un mode de réalisation préféré de l'invention, l'étape d'affichage consiste à afficher les compositions des produits déterminés lors de l'étape de détermination en les ordonnant par ordre de proximité entre la coloration théorique obtenue par chacun des produits de coloration et la coloration cible, préférentiellement de la coloration théorique la plus proche à la plus éloignée.

Dans cette étape d'affichage, et selon un mode de réalisation préféré de l'invention, une valeur représentative de l'écart entre la coloration cible et la coloration théorique obtenue par chaque produit de coloration est aussi affichée, ce qui permettra aux personnes qui vont choisir le produit de coloration qui sera élaboré d'évaluer la "différence" de coloration.

Cette valeur représentative doit être significative pour la personne qui effectue le choix final du produit de coloration à élaborer. Elle consiste donc en une valeur numérique de la variation $\Delta E^*$ du produit de coloration considérée, et selon une variante de réalisation de l'invention, cette valeur représentative consiste en un élément graphique, par exemple une "pastille" colorée de la couleur théorique obtenue par le produit de coloration ou bien elle consiste en une représentation graphique colorée à deux couleurs, une première couleur étant la coloration théorique obtenue par le produit de coloration considéré, et la deuxième couleur étant la coloration cible.

Lorsque la base de données 10 comporte aussi les troisièmes données 24 représentatives des caractéristiques économiques ou réglementaires, l'étape d'affichage consiste aussi à afficher ces troisièmes données 24 pour chacun des produits de coloration déterminés lors de l'étape de détermination.

Lorsque l'utilisateur d'un tel procédé est un coiffeur, il se peut qu'il ne dispose pas de la totalité des les éléments colorants enregistrés dans la base de données 10.

C'est pourquoi, selon une variante de l'invention, le procédé de détermination comporte une deuxième étape de saisie d'un ensemble d'éléments colorants qui sont les éléments colorants que possède l'utilisateur.

Cette deuxième étape de saisie peut aussi être utilisée par le fabricant de produits de coloration qui désire concevoir un produit de coloration en fonction de caractéristiques économiques par exemple en fonction du prix de revient des éléments colorants.

En fonction des compositions de produits de coloration affichées, il est alors possible de choisir le produit de coloration qui sera élaboré, le choix se faisant surtout en fonction de la coloration théoriquement obtenue, cependant, divers paramètres tels que les éléments colorants dont dispose l'utilisateur ou bien la complexité d'élaboration du produit de coloration peuvent entrer en considération.

Une fois que la composition du produit de coloration est choisie, l'utilisateur est alors apte à élaborer le produit de coloration choisi.

Pour cela, l'invention propose un procédé d'élaboration d'un produit de coloration dont la composition a été déterminée à partir du procédé de détermination décrit précédemment qui consiste à mélanger l'ensemble des éléments colorants cités lors de l'étape d'affichage et à les mélanger suivant les proportions citées lors de l'étape d'affichage.

L'utilisateur peut réaliser le dosage lui-même en dosant manuellement les éléments colorants nécessaires pour réaliser le produit de coloration, ou bien il peut avoir à sa disposition un dispositif automatique (non représenté) qui est relié à l'ordinateur 26 et qui est apte à doser chaque composant et à les mélanger en vue de la mise en oeuvre du produit de coloration.

Une fois le produit de coloration élaboré, le coiffeur, est apte à l'appliquer sur la chevelure à colorer pour obtenir la coloration voulue.

Selon un mode de réalisation particulier, les éléments colorants sont mélangés, de préférence au moment de l'emploi, à une composition contenant, dans un milieu approprié pour la coloration, au moins un agent oxydant, cet agent oxydant étant présent en une quantité suffisante pour développer une coloration. Le mélange obtenu est ensuite appliqué sur les fibres kératiniques. Après un temps de pose de 3 à 50 minutes environ, de préférence 5 à 30 minutes environ, les fibres kératiniques sont rincées, lavées au shampooing, rincées à nouveau puis séchées.

Les agents oxydants classiquement utilisés pour coloration d'oxydation des fibres kératiniques sont par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases. Le peroxyde d'hydrogène est particulièrement préféré.

La composition oxydante peut aussi renfermer divers adjuvants utilisés classiquement dans les compositions pour la teinture des cheveux et tels que définis précédemment.

Le pH de la composition oxydante renfermant l'agent oxydant est tel qu'après mélange avec les éléments colorants, le pH du produit de coloration résultant appliqué sur les fibres kératiniques varie de préférence entre 3 et 12 environ, et encore plus préférentiellement entre 5 et 11. Il peut être ajusté à la valeur désirée au

moyen d'agents acidifiants ou alcalinisants habituellement utilisés en teinture des fibres kératiniques et tels que définis précédemment.

La composition prête à l'emploi qui est finalement appliquée sur les fibres kératiniques peut se présenter sous des formes diverses, telles que sous forme de liquides, de crèmes, de gels ou sous toute autre forme appropriée pour réaliser une teinture des fibres kératiniques, et notamment des cheveux humains.

Selon une autre variante de réalisation de l'invention, l'étape d'affichage consiste à afficher les conditions d'application du produit de coloration, comme par exemple le temps de pose ou la température d'application, il est alors possible à l'utilisateur de connaître directement les conditions d'application du produit de coloration sélectionné.

## Revendications

1. Procédé de détermination de la composition d'un produit de coloration de fibres kératiniques comportant :

   - une étape de saisie d'au moins une donnée représentative d'une coloration cible à atteindre ; et
   - une étape de détermination, à partir d'une base de données, d'au moins un produit de coloration qui permet d'obtenir la coloration cible ou une coloration théorique qui s'écarte au plus de la coloration cible d'une valeur théorique prédéterminée, ou d'un nombre prédéterminé de produits qui permettent d'obtenir des colorations théoriques les plus proches de la coloration cible,

   **caractérisé en ce qu'**il comporte une étape d'affichage de la composition de chaque produit déterminé, identifiant le ou les éléments colorants nécessaires à leur réalisation, ainsi que les proportions respectives desdits éléments nécessaires.

2. Procédé selon la revendication précédente, **caractérisé en ce que** l'étape d'affichage consiste aussi à afficher, pour chaque produit déterminé, au moins une valeur représentative de l'écart entre la coloration théorique obtenue par le produit et la coloration cible.

3. Procédé selon la revendication 1, **caractérisé en ce que** l'étape d'affichage consiste aussi à afficher, pour chaque produit déterminé, un élément graphique coloré dont la coloration est la coloration théorique obtenue par le produit.

4. Procédé selon l'une quelconque des revendications précédentes, **caractérisé en ce que** l'étape d'affichage consiste à afficher les produits déterminés en les ordonnant par ordre de proximité entre la coloration théorique et la coloration cible.

5. Procédé selon l'une quelconque des revendications précédentes, **caractérisé en ce que** l'étape de détermination consiste à sélectionner un ou plusieurs produits présents dans la base de données et/ou à déterminer la composition d'un ou plusieurs produits par calcul numérique.

6. Procédé selon l'une quelconque des revendications précédentes, **caractérisé en ce qu'**au moins un élément colorant est constitué d'une base d'oxydation et éventuellement d'un coupleur d'oxydation.

7. Procédé selon l'une quelconque des revendications précédentes, **caractérisé en ce que** l'élément est constitué d'un colorant direct.

8. Procédé selon l'une quelconque des revendications précédentes, **caractérisé en ce qu'**il comporte en outre une étape de saisie d'au moins une caractéristique des cheveux à colorer.

9. Procédé selon la revendication précédente, **caractérisé en ce que** l'étape de saisie consiste à saisir au moins une donnée représentative de la couleur réelle des cheveux à colorer.

10. Procédé selon la revendication précédente, **caractérisé en ce qu'**il comporte une étape d'identification d'un ensemble réduit d'éléments colorants choisis parmi tous les éléments colorants contenus dans la base de données, et **en ce que** l'étape de détermination consiste à déterminer des produits de colorations formés d'un ou plusieurs éléments colorants dudit ensemble réduit.

11. Procédé de préparation d'un produit de coloration, comportant les étapes suivantes :

    i) déterminer la proportion d'un ou plusieurs éléments colorants entrant dans le produit de coloration à préparer à partir d'un procédé selon l'une quelconque des revendications précédentes ; et
    ii) préparer ledit produit de coloration en y incorporant le ou les éléments colorant déterminé lors de l'étape i) selon les proportions déterminé lors de ladite étape i).

12. Base de données susceptible d'être utilisée pour la mise en oeuvre d'au moins un des procédés selon l'une quelconque des revendications précédentes, **caractérisée en ce qu'**elle comporte ;

    a) des premières données pour identifier une

pluralité d'éléments colorants ;

b) des deuxièmes données comportant :

i) la concentration du ou des éléments colorants, choisis parmi ladite pluralité d'éléments colorants, et entrant dans la préparation d'une pluralité de produits de coloration ; et

ii) pour chacun desdits produits de coloration, la couleur obtenue lorsqu'ils sont appliqués selon des conditions prédéfinies.

**13.** Base de données selon la revendication précédente, **caractérisée en ce qu'**elle comporte des troisièmes données représentatives de caractéristiques économiques et/ou réglementaires relatives à au moins un desdits produits de coloration.

**14.** Procédé de constitution d'une base de données selon l'une des revendications 12 ou 13, **caractérisé en ce qu'**il comporte :

i) une étape d'enregistrement des premières données, et

ii) une étape d'enregistrement des deuxièmes données associées.

**15.** Procédé de constitution d'une base de données selon la revendication précédente, **caractérisé en ce qu'**il comporte une étape de mise à jour de la base de données en enregistrant les deuxièmes données relatives à au moins un nouveau produit de coloration.

**16.** Agencement pour la détermination de la composition d'un produit de coloration de fibres kératiniques en mettant en oeuvre un procédé selon l'une quelconque des revendications 1 à 11 comportant :

i) des moyens de saisie d'au moins une donnée représentative d'une coloration cible ;

ii) des moyens de calcul pour déterminer, à partir d'une base de données, au moins un produit qui permet d'obtenir la coloration cible ou une coloration théorique qui s'écarte au plus de la coloration cible d'une valeur théorique prédéterminée, ou d'un nombre prédéterminé de produits qui permettent d'obtenir des colorations théoriques les plus proches de la coloration cible ; et

iii) des moyens d'affichage de la composition de chaque produit déterminé, identifiant les éléments colorants nécessaires à leur élaboration, ainsi que les proportions respectives desdits éléments nécessaires.

**17.** Agencement selon la revendication précédente, **caractérisé en ce que** la base de données, et éventuellement les moyens de calcul, sont délocalisés relativement aux moyens de saisie et aux moyens d'affichage, les données entre au moins certains constituants de l'agencement pouvant transiter par un réseau informatique de type Internet.

**18.** Agencement selon la revendication précédente, **caractérisé en ce que** les moyens de saisie de la coloration cible comportent un colorimètre ou spectrocolorimètre électronique.

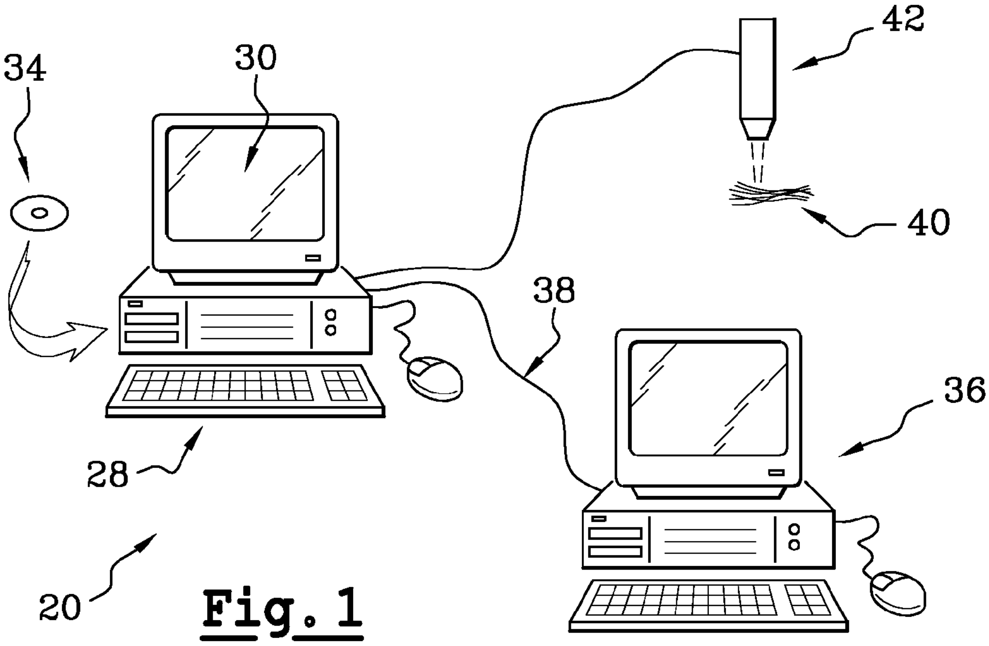

34
30
42
40
38
36
28
20


Fig. 1

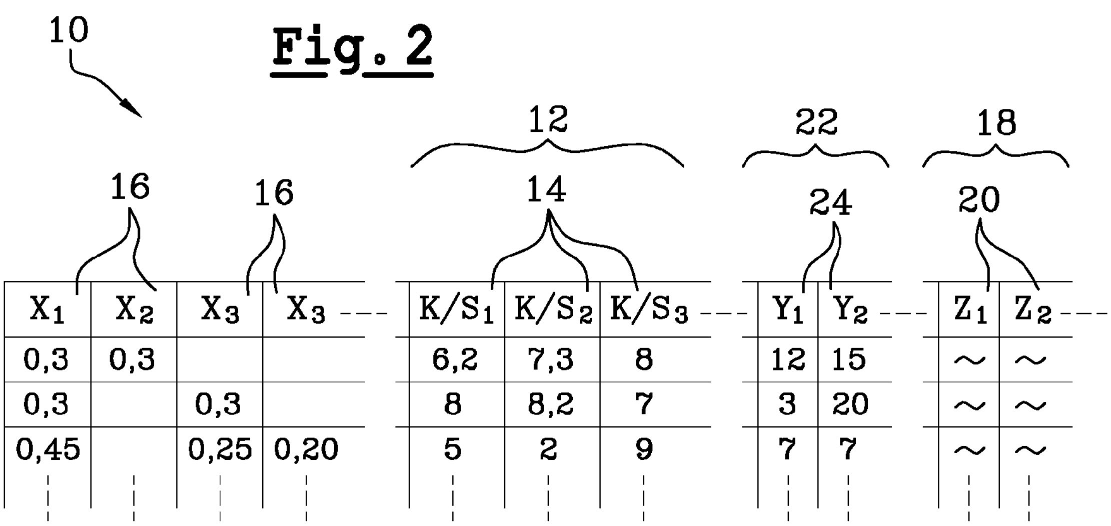

10
12
22
18
16
16
14
24
20
X1 X2 X3 X3
K/S1 K/S2 K/S3
Y1 Y2
Z1 Z2
0,3 0,3
6,2 7,3 8
12 15
0,3 0,3
8 8,2 7
3 20
0,45 0,25 0,20
5 2 9
7 7


Fig. 2

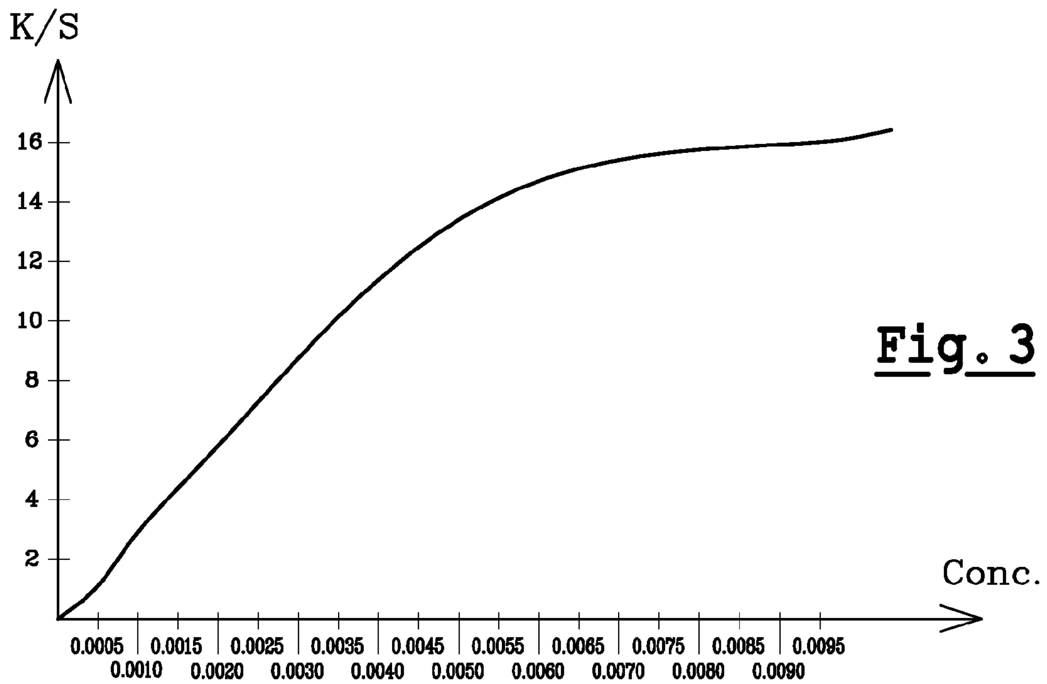
K/S
16
14
12
10
8
6
4
2
Conc.
0.0005
0.0010
0.0015
0.0020
0.0025
0.0030
0.0035
0.0040
0.0045
0.0050
0.0055
0.0060
0.0065
0.0070
0.0075
0.0080
0.0085
0.0090
0.0095

Fig. 3

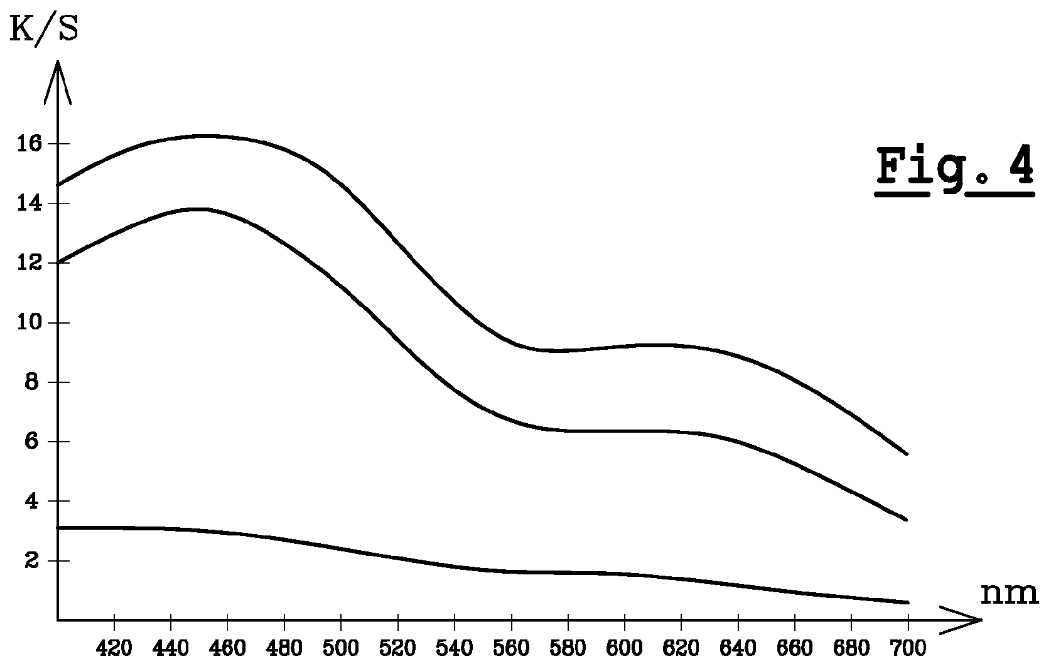
K/S
16
14
12
10
8
6
4
2
nm
420 440 460 480 500 520 540 560 580 600 620 640 660 680 700

Fig. 4

Office européen des brevets

# RAPPORT DE RECHERCHE EUROPEENNE

Numéro de la demande
EP 03 10 1986

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| X | US 4 813 000 A (WYMAN LARRY D ET AL) 14 mars 1989 (1989-03-14)<br>* exemples 1,2 *<br>* revendications 1,5,7 * | 1-18 | G01J3/46 |
| A | EP 1 147 722 A (KAO CORP) 24 octobre 2001 (2001-10-24)<br>* revendications 1-7 * | 1-18 | |
| A | US 2002/010556 A1 (MARAPANE SURESH BANDARA ET AL) 24 janvier 2002 (2002-01-24)<br>* revendications 13-18 * | 1-18 | |
| D,A | US 6 330 341 B1 (MACFARLANE ET AL) 11 décembre 2001 (2001-12-11)<br>* revendications 1-14 * | 1-18 | |

DOMAINES TECHNIQUES RECHERCHES (Int.Cl.7)

G01J
A61B

Le présent rapport a été établi pour toutes les revendications

| Lieu de la recherche | Date d'achèvement de la recherche | Examinateur |
|---|---|---|
| LA HAYE | 9 octobre 2003 | Rasmusson, R |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

**ANNEXE AU RAPPORT DE RECHERCHE EUROPEENNE RELATIF A LA DEMANDE DE BREVET EUROPEEN NO.** EP 03 10 1986

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

09-10-2003

| Document brevet cité au rapport de recherche | Date de publication | Membre(s) de la famille de brevet(s) | Date de publication |
|---|---|---|---|
| US 4813000 A | 14-03-1989 | AU 592877 B2 | 25-01-1990 |
| | | AU 7426787 A | 14-01-1988 |
| | | CA 1270951 A1 | 26-06-1990 |
| | | GB 2192455 A ,B | 13-01-1988 |
| | | JP 1928999 C | 12-05-1995 |
| | | JP 6052534 B | 06-07-1994 |
| | | JP 63153677 A | 27-06-1988 |
| | | NZ 220724 A | 29-08-1989 |
| EP 1147722 A | 24-10-2001 | EP 1147722 A1 | 24-10-2001 |
| | | CN 1339947 T | 13-03-2002 |
| | | WO 0132051 A1 | 10-05-2001 |
| | | JP 2001195568 A | 19-07-2001 |
| US 2002010556 A1 | 24-01-2002 | AU 6140401 A | 26-11-2001 |
| | | CA 2407457 A1 | 22-11-2001 |
| | | CN 1440503 T | 03-09-2003 |
| | | EP 1281054 A2 | 05-02-2003 |
| | | WO 0187245 A2 | 22-11-2001 |
| US 6330341 B1 | 11-12-2001 | US 6314372 B1 | 06-11-2001 |
| | | US 6067504 A | 23-05-2000 |
| | | US 5671735 A | 30-09-1997 |
| | | US 6308088 B1 | 23-10-2001 |
| | | US 6437863 B1 | 20-08-2002 |
| | | US 6157445 A | 05-12-2000 |
| | | US 6129664 A | 10-10-2000 |
| | | US 6128516 A | 03-10-2000 |

EPO FORM P0460

Pour tout renseignement concernant cette annexe : voir Journal Officiel de l'Office européen des brevets, No.12/82